# EUROPEAN PATENT SPECIFICATION

(11) **EP 0 721 359 B1**
(45) Date of publication and mention of the grant of the patent: **09.08.2000**
(21) Application number: 94928524.1
(22) Date of filing: 05.09.1994
(51) Int. Cl.: A61M 16/06

(54) **FACE MASK**
GESICHTSMASKE
MASQUE FACIAL

(30) Priority: 27.09.1993 SE 9303155
(43) Date of publication of application: 17.07.1996
(73) Proprietor: AstraZeneca AB, 151 85 Södertälje (SE)
(72) Inventor: WIDERSTRÖM, Carin, S-236 00 Höllviken (SE); KARLSSON, Jan, S-245 62 Hjärup (SE)
(86) International application number: PCT/SE94/00815
(87) International publication number: WO 95/09023

(56) References cited:
- WO-A-93/01854
- US-A- 3 042 035

## Description

The present invention relates to a face mask for infants adapted to be provided on an inhalator device having a body, said mask comprising an annular adaptor part which is to be connected to one end of the body of the inhalator device, a funnel-shaped face engaging portion which with its narrow end is joined to one end of the adaptor part, the face engaging portion being made of resilient material, the wide end of the face engaging portion being adapted to be brought in engagement with the face around the mouth and at least a part of the nose of the infant and being resilient to be adaptable to the face.

When dealing with bronchial diseases, such as asthma, among young children and infants, it is a problem to make them inhale the substances. When asthma makes its debute among young children such as infants from 8 months up to 2.5 years it is especially difficult to make the infant inhale the prescribed medical substances correctly in the proper way. It is also a want among the parents that the used devices should be as flexible as possible.

### Background art

It is known in the art to use stationary inhalation devices having expensive and complicated face masks which have shown to be very good also for infants. To use a stationary device could of course be inconvenient both for the infant and for the parents as they are bound to these stationary devices which are often placed in hospitals. As the inhalation capacity of an infant is limited, the administration will be time consuming, which of course also is inconvenient.

Devices are also known in the prior art, which are intended to be used by older children and sometimes these devices are used also for infants even when the result of the inhalation is not satisfying.

An infant has a limited lung capacity and the force of the breath is limited. This is even more apparent when the infant is suffering from asthma or other bronchial diseases. The known devices are all constructed to be used by older children who have large lung capacity and who can inhale more forcefully.

The known devices are provided with one way valves to avoid the air from the exhalation to penetrate into the spacer body. These valves require a certain inhalation flow to open and an infant is not capable to generate the requried inhalation flow to open the valve in the proper way.

EP 0 344 879 describes a face mask according to the preamble of claim 1 for use in connection with a device for inhalation of aerosols, where the face mask is connected to the spacer body and placed over the nose and mouth of the infant, see also EP 0 384 050.

WO 93/01854 describes an anaesthesia mask for infants, comprising the features of the preamble of claim 1.

The disadvantages with these face masks are that they present a rather large "dead space" between the valve in the spacer and the face mask,which requires a certain inhalation flow to insure that the substance to be inhaled does not stay in this "dead space", but reaches the lung area. Moreover, the shape of the face mask is not adapted to the face of an infant as it is round and the inhalation device is to be hold in a horisontal position which makes inhalation difficult for an infant. None of these two known devices is designed for the special needs of infants,who are suffering from bronchial diseases.

### The invention

The face mask according to the present invention is intended to be used by infants and young children up to the age of about 2.5 years. In the following the word infant will be used to describe both an infant and an young child up to this age.

The face mask according to the invention is intended to be used in connection with a metered-dose aerosol inhalator for inhalation of aerosols, but could also be used together with any other inhalation device. A metered-dose aerosol inhalation device includes a holder for a medicament dispenser and an extended body, which in the technical field of inhalators normally is called a "spacer" or inhalation chamber, having an outlet provided at the end remote from the dispenser. Adjacent to the outlet an inhalation/exhalation valve is provided, preferably a so called one way valve, and at the outlet opening a mouthpiece is provided. When such a device is used by older children or adults the mouthpiece is inserted between the teeth and the lips are closed around the mouthpiece. It is however not possible for an infant to hold such a mouthpiece between its lips and therefore the inhalation device must be provided with a face mask when used by infants.

It is an object of the invention to provide a face mask for infants which overcomes the problems with the known face masks.

This and other objects are achieved by providing a face mask according to the invention with the characterising features of claim 1, where the free edge of the wide end of the face engaging part is provided substantially in a plane which forms an angle of about 10° - 25° with a plane perpendicular to the extended geometrical axis of the adaptor part.

This inclination of the plane of the wide end of the face engaging portion makes it possible to decrease the dead space between the inhalation/exhalation valve and the mouth of the infant and makes it natural for the user to hold the inhalation device in an inclined position in relation to the infant's face in a manner which corresponds to the inclination of a baby feeding-bottle during feeding. This inclination will contribute to keeping the one way valve in the mouthpiece end of the spacer body of the inhalator opened. This is important because thereby resistance during inhalation is decreased. Through this inclination the dead space between the valve of the spacer and the mask could be decreased and kept as small as possible. Moreover the inclination of the mask and the spacer body makes it more comfortable for both the infant and the parent and as a result of the inclination the inhalation is improved.

Other prefered embodiments of the face mask according to the invention are defined in the dependent claims.

### Detailed description of the invention

One preferred embodiment of the invention will now be described with reference to the drawings where
Fig. 1 shows a sectional side view of a face mask according to the invention,
Fig. 2 shows the face mask as viewed from above in Fig. 1,
Fig. 3 is a perspective view of the face mask, and
Fig. 4 shows a sectional side view of a second embodiment of the face mask.

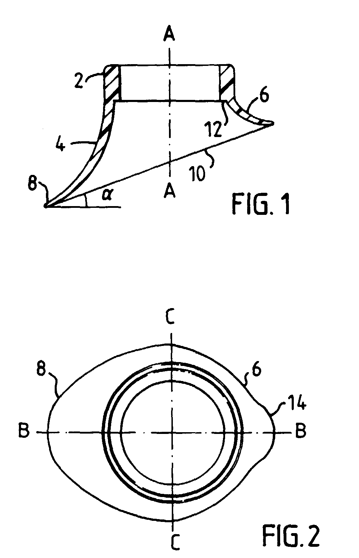

In Figure 1 the face mask is shown in a side view. The face mask consists of two parts, a first part, the adaptor part 2, which is intended to be connected to the end of the spacer body of the inhalator (not shown) and a second part, the face engaging portion 4 which is intended to be held against an infants face during inhalation.

The adaptor part 2 is annular and preferably circular and has a length of preferably 12 mm. The thickness of the material in the adaptor part 2 is preferably about 5 mm.

The face engaging portion 4 is funnel-shaped and widens towards the free edge 10. The sides of the face engaging portion 4 are different in length, a short side 6 and a long side 8, as a consequence of the fact that the free edge 10 of the wide end of the face engaging portion is provided substantially in a plane which forms an angle α of about 10° - 25°, preferably 15° - 20° with the plane perpendicular to the geometrical axis A-A of the adaptor part 2.

The circular adaptor part 2 smoothly merges with the face engaging portion 4 without any sharp edges or seams. At the junction between the two parts 2 and 4 of the face mask a shoulder 12 is provided. This shoulder is placed at the point where the thickness of the adaptor part 2 decreases from preferably about 5 to about 2 - 3 mm, which is the thickness of the face engaging portion 4 at its base. The thickness of the material in the face engaging portion 4 decreases from about 2 - 3 mm at the junction with the adaptor part 2 to preferably about 0.5 mm at the free edge 10.

On the short side 6 of the face engaging portion 4 a nose engaging part 14 is provided which is meant to at least partly cover the nose of the infant during inhalation. The length of the short side 6 is preferably about 20 mm including the adaptor part 2 and the length of the long side 8 is preferably about 48 mm including the adaptor part 2.

The outer contour of the walls of the funnel-shaped face engaging portion 4 is concave and preferably forms an arc of an circle where the radius of the long side 8 is preferably about 40 mm and the radius of the short side 6 is preferably about 16 mm. The inner contour of the walls is convex and preferably also forms an arc of an circle where the radius of the long side 8 is preferably about 38 mm and the radius of the short side 6 is preferably about 20 mm.

As the different radii of the outer and the inner contours respectively have different centre points there will be a gradual decrease in thickness of the walls.
The funnel-shaped face engaging portion 4 has an oval form to adapt to the form of an infant's face. The ratio between the major axis (B-B) and the minor axis (C-C) is thereby 1.2 - 1.4, see Fig 2.

The free edge 10 and the adjacent part of the face engaging portion 4 are comperatively thin and therefore very flexible. When the face mask is placed around the mouth and at least part of the nose of art infant the outer part of the face engaging portion can therefore well adapt to the face of the infant so that if required the mask can seal against the face in an air tight manner. This is especially important when the substance to be inhaled comprises stereoids or other substances with which the eyes must not come into contact.

As can be seen in Fig. 2 the nose engaging part 14 can be shaped as a substantial semi-circular protrusion extending from the edge of the short side 6. With this protrusion the adaption to the nose of the infant is improved.

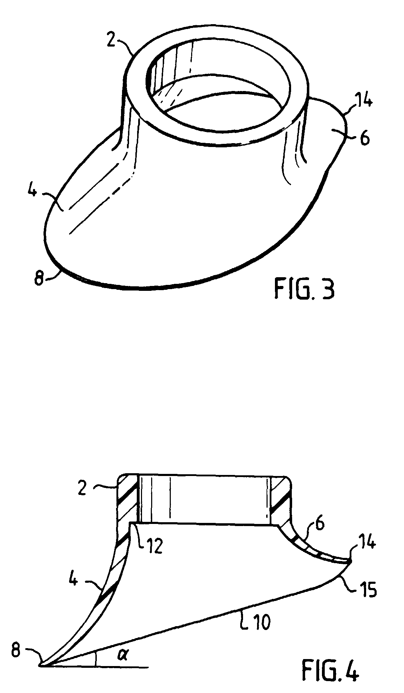

The nose part 14 could also be provided with a more inclined edge to better adapt to the shape of an infants nose. The outer edge of the nose part is thereby drawn backwards to the adaptor part 2 as shown in Fig. 4.

The mask according to the invention is preferably made of thermoplastic material or other rubber-like material being soft and resilient such as thermoplastic elastomers or silicon-like materials but of course other materials having similar properties could be used.

In the above reference has been made to treatment of asthmatic diseases by inhalation of suitable medical substances, but it is apperent that the face mask according to the invention also could be used for inhalation of other medical substances, such as for example anesthetics.

Reference is also made to the use of the face mask together with metered-dose aerosol inhalators, but the face mask could also be used together with any kind of inhalators provided that they have an adaptor part at their mouthpiece end adapted to fit to the adaptor part of the face mask.

### Possible modifications

The face mask could of course be modified within the scope of the appended claims.

Thus the inside of the adaptor part, which in the above described embodiment is provided with a shoulder in the transition between the adaptor part and the face engaging portion, could smoothly merge into the face engaging part.

Although, it is not necessary as the material and the thin thickness of the edge of the face mask makes it possible for the exhalation air to escape from the face mask and the inhalator, the face engaging portion could be provided with an exhalation hole to make the escape of the exhaled air easier during the inhalation treatment.

## Claims

1. A face mask for an infant, comprising:
an annular adaptor part (2) having first and second ends, the first end of which is adapted for connection to an inhalation device; and
a face engaging part (4) comprising an outwardly flaring wall section having a narrow end connected to the second end of the adaptor part (2) and a wide end having a free edge (10) adapted to engage an area around the mouth and at least part of the nose of an infant, the free edge (10) of the wide end of the face engaging part (4) being resilient so as to be conformable to the area around the mouth and the at least part of the nose of the infant;
characterized in that the face engaging part (4) includes a short side (6) which defines a nose engaging portion (14) adapted to be positioned against the nose of the infant and a long side (8) adapted to be positioned below the mouth of the infant and in that the free edge (10) of the wide end of the face engaging part (4) defines and is disposed substantially entirely in a plane which encloses an angle of from 10 to 25° with a plane perpendicular to the extended longitudinal axis of the adaptor part (2).

2. The face mask of claim 1, wherein the outer surface of the face mask includes a smooth surface at the junction between the outer surfaces of the adaptor part (2) and the face engaging part (4).

3. The face mask of claim 1 or 2, wherein the inner surface of the face mask includes a shoulder (12) at the junction between the inner surfaces of the adaptor part (2) and the face engaging part (4).

4. The face mask of any of claims 1 to 3, wherein the outer surface of the face engaging part (4) is concave.

5. The face mask of claim 4, wherein the outer surface of the face engaging part (4) in longitudinal section describes an arc of a circle.

6. The face mask of any of claims 1 to 5, wherein the inner surface of the face engaging part (4) is convex.

7. The face mask of claim 6, wherein the inner surface of the face engaging part (4) in longitudinal section describes an arc of a circle.

8. The face mask of any of claims 1 to 7, wherein the free edge (10) of the wide end of the face engaging part (4) describes an oval.

9. The face mask of claim 8, wherein the ratio between the major axis (B-B) and the minor axis (C-C) of the oval is from 1.2 to 1.4

10. The face mask of any of claims 1 to 9, wherein the thickness of the wall section of the face engaging part (4) decreases gradually towards the free edge (10) of the wide end thereof.

11. The face mask of any of claims 1 to 10, wherein the nose engaging portion (14) of the face engaging part (4) includes a protrusion (15).

12. The face mask of claim 11, wherein the protrusion (15) is substantially semi-circular.

13. The face mask of any of claims 1 to 12, wherein the face mask is a single unitary member.

14. The face mask of any of claims 1 to 13, wherein the face mask is composed of a thermoplastics material.

## Patentansprüche

1. Gesichtsmaske für einen Säugling, die Folgendes umfasst : einen ringförmigen Anschlussteil (2) mit einem ersten und zweiten Ende, wobei das erste Ende an ein Inhalationsgerät angeschlossen werden kann, und einen das Gesicht in Eingriff nehmenden Teil (4), der einen nach außen kelchenden Wandabschnitt mit einem schmalen, an das zweite Ende des Anschlussteils (2) angeschlossenen Ende und einem weiten Ende mit einer freien Kante (10) umfasst, die einen Bereich um den Mund und mindestens einen Teil der Nase eines Säuglings in Eingriff nehmen kann, wobei die freie Kante (10) des weiten Endes des das Gesicht in Eingriff nehmenden Teils (4) so elastisch ist, dass sie sich an den Bereich um den Mund und mindestens den Teil der Nase des Säuglings anpasst, dadurch gekennzeichnet, dass der das Gesicht in Eingriff nehmende Teil (4) eine kurze Seite (6), die einen die Nase in Eingriff nehmenden Abschnitt (14) definiert, der an der Nase des Säuglings positioniert werden kann, und eine lange Seite (8), die unter dem Mund des Säuglings positioniert werden kann, aufweist und dass die freie Kante (10) des weiten Endes des das Gesicht in Eingriff nehmenden Teils (4) eine Ebene definiert, und im Wesentlichen vollständig in dieser angeordnet ist, die mit einer zur verlängerten Längsachse des Anschlussteils (2) senkrechten Ebene einen Winkel von 10 bis 25° einschließt.

2. Gesichtsmaske nach Anspruch 1, bei der die Außenfläche der Gesichtsmaske am Übergang zwischen der Außenfläche des Anschlussteils (2) und der Außenfläche des das Gesicht in Eingriff nehmenden Teils (4) eine glatte Fläche aufweist.

3. Gesichtsmaske nach Anspruch 1 oder 2, bei der die Innenfläche der Gesichtsmaske am Übergang zwischen der Innenfläche des Anschlussteils (2) und der Innenfläche des das Gesicht in Eingriff nehmenden Teils (4) einen Absatz (12) aufweist.

4. Gesichtsmaske nach einem der Ansprüche 1 bis 3, bei der die Außenfläche des das Gesicht in Eingriff nehmenden Teils (4) konkav ist.

5. Gesichtsmaske nach Anspruch 4, bei der die Außenfläche des das Gesicht in Eingriff nehmenden Teils (4) im Längsschnitt einen Kreisbogen beschreibt.

6. Gesichtsmaske nach einem der Ansprüche 1 bis 5, bei der Innenfläche des das Gesicht in Eingriff nehmenden Teils (4) konvex ist.

7. Gesichtsmaske nach Anspruch 6, bei der die Innenfläche des das Gesicht in Eingriff nehmenden Teils (4) im Längsschnitt einen Kreisbogen beschreibt.

8. Gesichtsmaske nach einem der Ansprüche 1 bis 7, bei der die freie Kante (10) des weiten Endes des das Gesicht in Eingriff nehmenden Teils (4) ein Oval beschreibt.

9. Gesichtsmaske nach Anspruch 8, bei der das Verhältnis zwischen der Hauptachse (B-B) und der Nebenachse (C-C) des Ovals zwischen 1,2 und 1,4 liegt.

10. Gesichtsmaske nach einem der Ansprüche 1 bis 9, bei der die Dicke des Wandabschnitts des das Gesicht in Eingriff nehmenden Teils (4) zur freien Kante (10) seines weiten Endes hin allmählich abnimmt.

11. Gesichtsmaske nach einem der Ansprüche 1 bis 10, bei der der die Nase in Eingriff nehmende Abschnitt (14) des das Gesicht in Eingriff nehmenden Teils (4) eine Auskragung (15) aufweist.

12. Gesichtsmaske nach Anspruch 11, bei der die Auskragung (15) im wesentlichen halbkreisförmig ist.

13. Gesichtsmaske nach einem der Ansprüche 1 bis 12, bei der es sich um ein einstückiges Einzelglied handelt.

14. Gesichtsmaske nach einem der Ansprüche 1 bis 13, die aus einem thermoplastischen Material besteht.

## Revendications

1. Masque facial pour nourrisson, comprenant :
une partie d'adaptateur annulaire (2) ayant des première et deuxième extrémités, dont la première extrémité est adaptée pour être connectée à un dispositif d'inhalation ; et
une partie d'engagement du visage (4) comprenant une section de paroi évasée vers l'extérieur ayant une extrémité étroite connectée à la deuxième extrémité de la partie d'adaptateur (2) et une extrémité large ayant un bord libre (10) adapté pour s'engager avec une zone autour de la bouche et au moins une partie du nez d'un nourrisson, le bord libre (10) de l'extrémité large de la partie d'engagement du visage (4) étant élastique de manière à epouser la zone autour de la bouche et au moins une partie du nez du nourrisson ;
caractérisé en ce que la partie d'engagement du visage (4) comporte un côté court (6) qui définit une portion d'engagement du nez (14) adaptée pour être positionnée contre le nez du nourrisson et un côté long (8) adapté pour être positionné sous la bouche du nourrisson, et en ce que le bord libre (10) de l'extrémité large de la partie d'engagement du visage (4) définit et est disposée substantiellement entièrement dans un plan qui forme un angle de 10 à 25° avec un plan perpendiculaire à l'axe longitudinal prolongé de la partie d'adaptateur (2) .

2. Masque facial selon la revendication 1, dans lequel la surface externe du masque facial comporte une surface lisse au niveau de la jonction entre les surfaces extérieures de la partie d'adaptateur (2) et la partie d'engagement du visage (4) .

3. Masque facial selon la revendication 1 ou 2, dans lequel la surface interne du masque facial comporte un épaulement (12) au niveau de la jonction entre les surfaces internes de la partie d'adaptateur (2) et de la partie d'engagement du visage (4) .

4. Masque facial selon l'une quelconque des revendications 1 à 3, dans lequel la surface extérieure de la partie d'engagement du visage (4) est concave.

5. Masque facial selon la revendication 4, dans lequel la surface extérieure de la partie d'engagement du visage (4) en coupe longitudinale décrit un arc de cercle.

6. Masque facial selon l'une quelconque des revendications 1 à 5, dans lequel la surface interne de la partie d'engagement du visage (4) est convexe.

7. Masque facial selon la revendication 6, dans lequel la surface interne de la partie d'engagement du visage (4) en coupe longitudinale décrit un arc de cercle.

8. Masque facial selon l'une quelconque des revendications 1 à 7, dans lequel le bord libre (10) de l'extrémité large de la partie d'engagement du visage (4) décrit un ovale.

9. Masque facial selon la revendication 8, dans lequel le rapport entre le grand axe (B-B) et le petit axe (C-C) de l'ovale est compris entre 1,2 et 1,4.

10. Masque facial selon l'une quelconque des revendications 1 à 9, dans lequel l'épaisseur de la section de paroi de la partie d'engagement du visage (4) diminue progressivement vers le bord libre (10) de l'extrémité large de celle-ci.

11. Masque facial selon l'une quelconque des revendications 1 à 10, dans lequel la portion d'engagement du nez (14) de la partie d'engagement du visage (4) comporte une saillie (15).

12. Masque facial selon la revendication 11, dans lequel la saillie (15) est substantiellement semi-circulaire.

13. Masque facial selon l'une quelconque des revendications 1 à 12, dans lequel le masque facial est un élément unique d'une pièce.

14. Masque facial selon l'une quelconque des revendications 1 à 13, dans lequel le masque facial se compose d'un matériau thermoplastique.
